# EUROPEAN PATENT APPLICATION

(11) **EP 3 546 013 A1**
(43) Date of publication of application: **02.10.2019**
(21) Application number: 16856461.5
(22) Date of filing: 22.11.2016
(51) Int. Cl.: A61M 25/10

(54) **BALLOON CATHETER**

(71) Applicant: Asahi Intecc Co., Ltd., Seto-shi, Aichi 489-0071 (JP)
(72) Inventor: TSUKAMOTO Toshihiko, Nagoya-shi Aichi 463-0024 (JP); KAWAGUCHI Keisuke, Nagoya-shi Aichi 463-0024 (JP); YAMANAKA Nobuyoshi, Nagoya-shi Aichi 463-0024 (JP); KANEKO Yoshiki, Nagoya-shi Aichi 463-0024 (JP)
(74) Representative: Winter, Brandl, Fürniss, Hübner, Röss, Kaiser, Polte - Partnerschaft mbB
(86) International application number: PCT/JP2016/084550
(87) International publication number: WO 2018/096572

(57) **Abstract**

[Problems to be Solved] Provided is a balloon catheter comprising a balloon. The balloon allows restraint-free arrangement of an irregular region on the balloon for quickly inserting the balloon into a stenosis or stricture or an obstructed part while the balloon is in a contracted state and for securely fixing the balloon within the stenosis or stricture or the obstructed part while the balloon is in an expanded state; can be easily produced; and can be folded without restraint, namely without requiring differentiation between the irregular region and the rest.

[Solution] A balloon catheter 1 comprises a balloon 3, and the balloon comprises a base portion 3x having an irregular region P on the surface and a coating agent 3d coating the surface of the base portion 3x, with the following configuration: before the balloon 3 is expanded, the surface of the balloon 3 is smooth and flat due to the presence of the coating agent 3d; and while the balloon 3 is in an expanded state, the surface of the balloon 3 is irregular due to the presence of the irregular region P of the base portion 3x.

## Description

### Field

The present invention relates to a balloon catheter for insertion into a stenosis or stricture or an obstructed part that is formed within an internal lumen such as a blood vessel, a bile duct, or a pancreatic duct for the purpose of dilating the stenosis or stricture or the obstructed part and thereby ensuring the flow of blood, bile (biliary fluid), pancreatic juice, or the like. Background

A stenosis or stricture or an obstructed part that is formed within an internal lumen such as a blood vessel, a bile duct, or a pancreatic duct interrupts the flow of blood, bile (biliary fluid), pancreatic juice, or the like. Conventionally, there are methods widely used for treating the stenosis or stricture or the obstructed part, which use a balloon catheter.

A balloon catheter primarily comprises an expandable balloon, an outer tube fixed to the proximal end of the balloon, and an inner tube accommodated within the interior of both the balloon and the outer tube. A guidewire is inserted into the inner tube and then travels through the inner tube. An expansion lumen is interposed between the outer tube and the inner tube, and through this expansion lumen, liquid (such as a contrast medium and physiological saline) for expanding the balloon travels.

In a typical balloon catheter, the balloon is folded around the outer circumference of the inner tube while the catheter is being inserted into an internal lumen such as a blood vessel, a bile duct, or a pancreatic duct and then when the catheter reaches a stenosis or stricture or an obstructed part, the balloon is expanded within the stenosis or stricture or the obstructed part.

During insertion of the catheter with the balloon folded around the outer circumference of the inner tube, the frictional force between the balloon and the internal lumen is preferably as small as possible for quick insertion of the balloon into the stenosis or stricture or the obstructed part.

While the balloon is in an expanded state within the stenosis or stricture or the obstructed part, the frictional force between the balloon and the stenosis or stricture or the obstructed part is preferably as great as possible for securely fixing the balloon within the stenosis or stricture or the obstructed part.

For example, Patent Literature 1 describes a balloon catheter that has a low coefficient of sliding friction while the balloon is in a contracted state and a high coefficient of sliding friction while the balloon is in an expanded state.

More specifically, this balloon catheter has the following configuration: while the balloon is in a contracted state, a region with a low coefficient of friction stretches over the entire surface of the balloon and a region with a high coefficient of friction is enclosed within the folds of the balloon; and while the balloon is in an expanded state, the region with a high coefficient of friction comes out of the folds of the balloon to stretch over the surface of the balloon, allowing fixation of the balloon within a stenosis or stricture or an obstructed part.

However, the balloon catheter described in Patent Literature 1 requires a complicated balloon-producing process in order to provide the region with a low coefficient of friction and the region with a high coefficient of friction in an arrangement in which these two regions appear alternately on the circumference of a cross section of the balloon and also in order to have the region with a low coefficient of friction stretch over the entire surface of the balloon and enclose the region with a high coefficient of friction within the folds of the balloon while the balloon is in a contracted state.

The balloon catheter described in Patent Literature 1 has another problem: because the region with a low coefficient of friction and the region with a high coefficient of friction need to be alternately arranged on the circumference of a cross section of the balloon as described above, freedom in the arrangement of the region with a low coefficient of friction and the region with a high coefficient of friction is limited.

### Citation List

### Patent Literature

Patent Literature 1: Japanese Patent Application Publication No. 2005-224635 (JP 2005-224635 A)

### Summary

### Technical Problem

The present invention has been devised based on the above circumstances. An object of the present invention is to provide a balloon catheter comprising a balloon. The balloon allows restraint-free arrangement of a region with a high coefficient of friction (hereinafter, also called irregular region) on the balloon for quickly inserting the balloon into a stenosis or stricture or an obstructed part while the balloon is in a contracted state and for securely fixing the balloon within the stenosis or stricture or the obstructed part while the balloon is in an expanded state; can be easily produced; and can be folded without restraint, namely without requiring differentiation between the irregular region and the rest.

### Solution to Problem

In order to achieve the object above, embodiment 1 of the present invention provides a balloon catheter comprising a balloon, the balloon comprising:
a base layer having an irregular region on a surface thereof; and
a coating layer coating the surface of the base layer, wherein
before the balloon is expanded, the surface of the balloon is smooth and flat due to the presence of the coating layer, and
while the balloon is in an expanded state, the surface of the balloon is irregular due to the presence of the irregular region of the base layer.

Embodiment 2 of the present invention provides the balloon catheter according to embodiment 1 of the present invention, wherein the irregular region of the base layer stretches over the entire circumference of a cross section vertical to the long-axis direction.

Embodiment 3 of the present invention provides the balloon catheter according to embodiment 1 or embodiment 2 of the present invention, wherein upon expansion of the balloon, the surface of the coating layer breaks and thereby the surface of the balloon becomes irregular.

### Advantageous Effects of Invention

A balloon catheter according to embodiment 1 of the present invention comprises a balloon, and the balloon comprises a base layer having an irregular region on the surface and a coating layer coating the surface of the base layer, with the following configuration: before the balloon is expanded, the surface of the balloon is smooth and flat due to the presence of the coating layer; and while the balloon is in an expanded state, the surface of the balloon is irregular due to the presence of the irregular region of the base layer. With this configuration, the balloon allows restraint-free arrangement of the irregular region on the balloon and can be easily produced.

In addition, the balloon can be folded without restraint, namely without requiring differentiation between the irregular region and the rest.

A balloon catheter according to embodiment 2 of the present invention is the balloon catheter according to embodiment 1 of the present invention, wherein the irregular region of the base layer stretches over the entire circumference of a cross section vertical to the long-axis direction. With this configuration, the balloon catheter according to embodiment 2 of the present invention has an effect of allowing easy fixation of the balloon to an affected area regardless of where the affected area is located, in addition to the effect of the balloon catheter according to embodiment 1 of the present invention.

A balloon catheter according to embodiment 3 of the present invention is the balloon catheter according to embodiment 1 or embodiment 2 of the present invention, wherein upon expansion of the balloon, the surface of the coating layer breaks and thereby the surface of the balloon becomes irregular. Because of this configuration, fixation of the balloon to an affected area can become even easier.

### Brief Description of Drawings

FIG. 1 is a schematic, overall view of a balloon catheter according to the first embodiment of the present invention (the balloon in the figure is in a contracted state).
FIG. 2 is a sectional view of the balloon in FIG. 1 in a slightly expanded state, taken from line A-A.
FIG. 3 is a sectional view of the balloon in FIG. 1 that is folded differently and slightly expanded, taken from line A-A.
FIG. 4 is a schematic, overall view of the balloon catheter according to the first embodiment (the balloon in this figure is in an expanded state).
FIG. 5 illustrates an irregular region of the balloon catheter according to the first embodiment (the balloon in this figure is in an expanded state).
FIG. 6 is a sectional view taken from line B-B of FIG. 5.
FIG. 7 illustrates an irregular region of a balloon catheter according to the second embodiment (the balloon in this figure is in an expanded state).
FIG. 8 is a sectional view taken from line C-C of FIG. 7.
FIG. 9 illustrates an irregular region of a balloon catheter according to the third embodiment (the balloon in this figure is in an expanded state).
FIG. 10 is a sectional view taken from line D-D of FIG. 9.

### Description of Embodiments

Embodiments of the present invention mentioned above will be described with reference to drawings.

### (First embodiment)

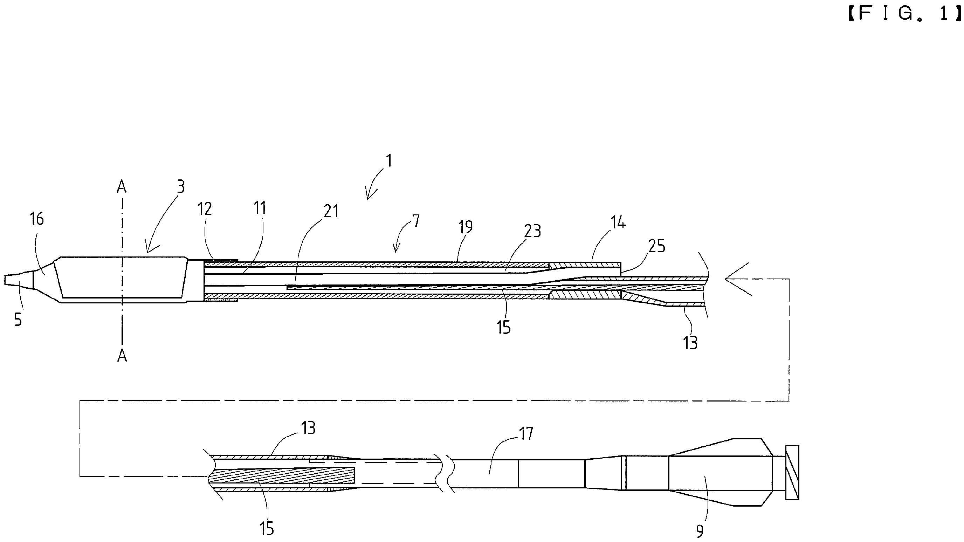

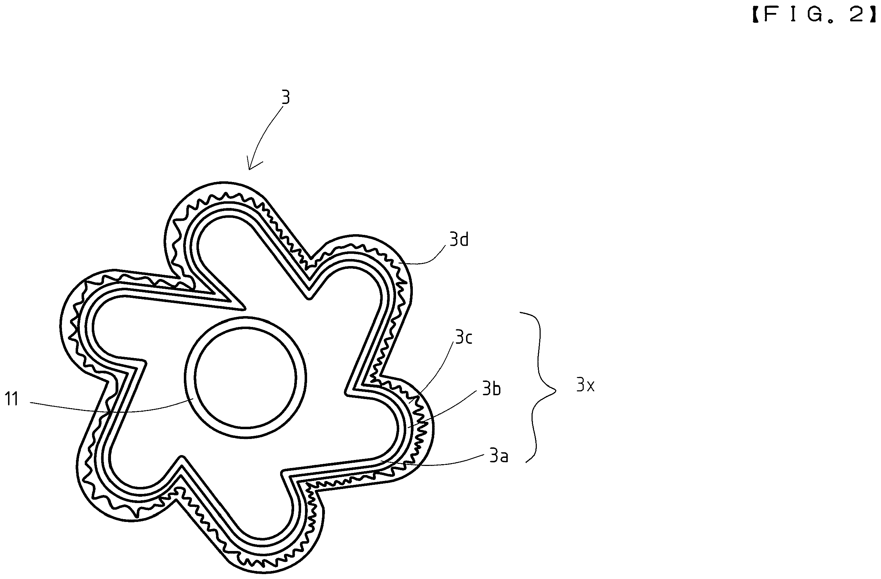

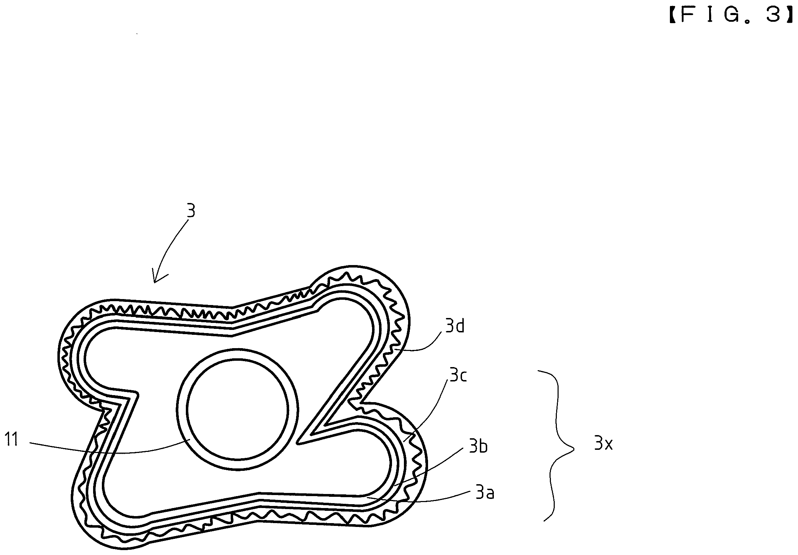

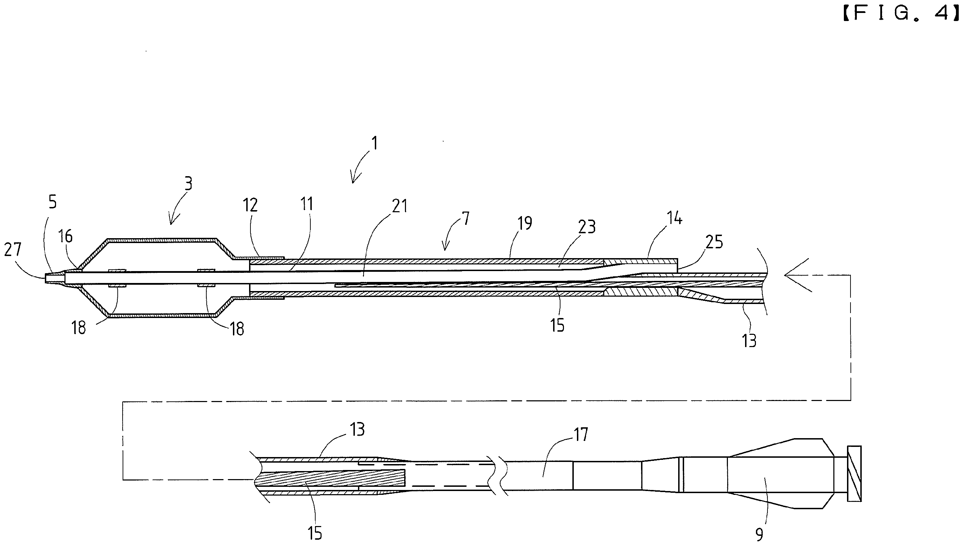

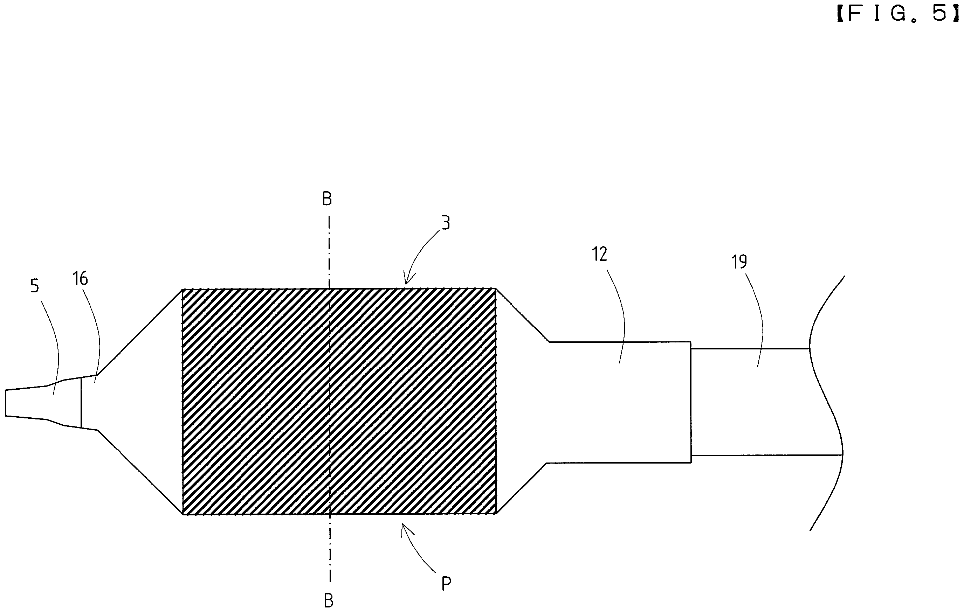

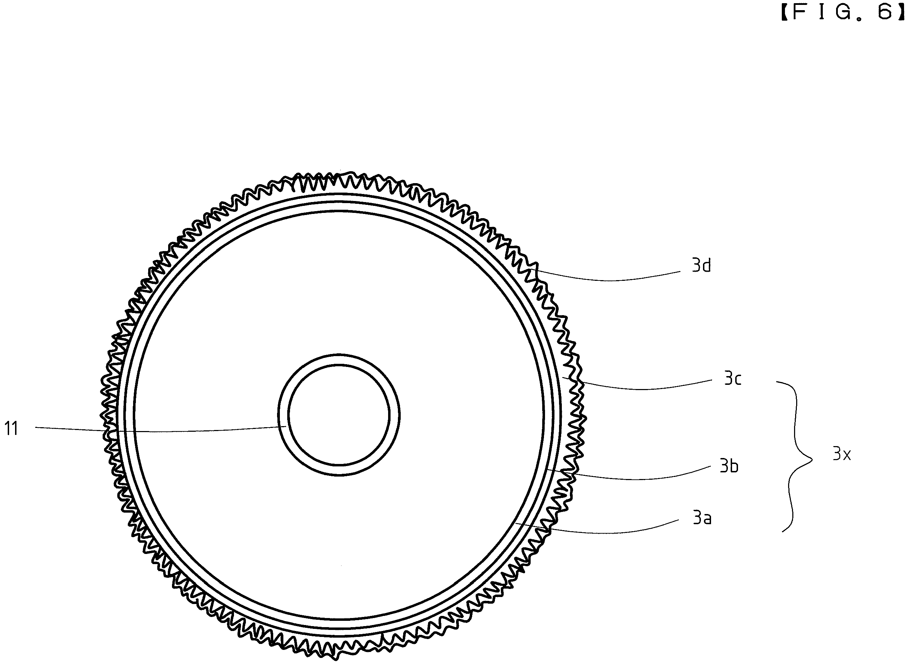

FIG. 1 is a schematic, overall view of a balloon catheter according to the first embodiment of the present invention (the balloon in the figure is in a contracted state); FIG. 2 is a sectional view of the balloon in FIG. 1 in a slightly expanded state, taken from line A-A; FIG. 3 is a sectional view of the balloon in FIG. 1 that is folded differently and slightly expanded, taken from line A-A; FIG. 4 is a schematic, overall view of the balloon catheter according to the first embodiment (the balloon in the figure is in an expanded state); FIG. 5 illustrates an irregular region of the balloon catheter according to the first embodiment (the balloon in the figure is in an expanded state); and FIG. 6 is a sectional view taken from line B-B of FIG. 5.

A balloon catheter 1 in FIG. 1 is used, for example, for treating a stenosis or stricture or an obstructed part that is formed within an internal lumen such as a blood vessel, a bile duct, or a pancreatic duct. The balloon catheter 1 primarily comprises a balloon 3, an outer tube 7, a connector 9, an inner tube 11, and a front-end tip 5. The balloon 3 in FIG. 1 is in a contracted state.

The balloon 3 for dilating a stenosis or stricture or an obstructed part is a resin component. A front-end-fitting member 16 of the balloon 3 is fixed to the front end of the inner tube 11 and to the front-end tip 5. A proximal-end-fitting member 12 of the balloon 3 is fixed to the front end of the outer tube 7.

In this embodiment, the front-end-fitting member 16 of the balloon 3 is fixed in an arrangement in which it covers the front-end tip 5 and the front end of the inner tube 11 (see FIG. 4). Alternatively, the front-end-fitting member 16 of the balloon 3 may be fixed and interposed between the front end of the inner tube 11 and the front-end tip 5.

In this embodiment, the proximal-end-fitting member 12 of the balloon 3 is fixed to the outer circumference of the front end of the outer tube 7. Alternatively, the proximal-end-fitting member 12 of the balloon 3 may be fixed to the inner circumference of the front end of the outer tube 7.

The outer tube 7 is a tubular component constituting an expansion lumen 23 that feeds liquid for expanding the balloon 3 such as a contrast medium and physiological saline. The outer tube 7 is composed of, from the front-end side, an outer-tube front end 19, a guidewire port 14, an outer-tube middle portion 13, and an outer-tube proximal end 17. Each of the outer-tube front end 19 and the outer-tube middle portion 13 is a tube made of a resin such as a polyamide, a polyamide elastomer, a polyolefin, a polyester, or a polyester elastomer. The guidewire port 14 is where the outer-tube front end 19, the outer-tube middle portion 13, and the inner tube 11 are fixed to each other.

The outer-tube front end 19 accommodates the inner tube 11 inserted thereinto. Between the outer-tube front end 19 and the inner tube 11, the expansion lumen 23 described above is disposed.

The outer-tube proximal end 17 is a tubular metal component, called a hypotube. The front end of the outer-tube proximal end 17 is accommodated within and fixed to the proximal end of the outer-tube middle portion 13. The proximal end of the outer-tube proximal end 17 has the connector 9 attached thereto. From an indeflator (not shown, attachable to the connector 9), liquid for expanding the balloon 3 such as a contrast medium and physiological saline is fed, which travels through the expansion lumen 23 to reach the balloon 3 to expand it.

The material of the outer-tube proximal end 17 is not particularly limited and may be a super-elastic alloy such as stainless steel (SUS304) or Ni-Ti alloy.

Accommodated in the interior of the inner tube 11 is a guidewire lumen 21 into which a guidewire is inserted. The proximal end of the inner tube 11 is fixed to the guidewire port 14 of the outer tube 7 to form a proximal-end-side guidewire port 25.

The front end of the inner tube 11 is fixed to the front-end tip 5 and to the front-end-fitting member 16 of the balloon 3. The front-end tip 5 is a tapered component with its outer diameter gradually decreasing toward the front end, made of a flexible resin. The resin as a material of the front-end tip 5 is not particularly limited and may be a polyurethane or a polyurethane elastomer, for example.

The front-end tip 5 is a tubular component fixed to the front end of the guidewire lumen 21 and having a front-end-side guidewire port 27 on its front end (see FIG. 4) .

So as to enable tracking of the location of the balloon 3 under radiation, the inner tube 11 comprises two radiopaque markers 18 (see FIG. 4) in the interior of the balloon 3.

To the inner circumference of the front end of the outer-tube proximal end 17, a reinforcing member 15 is attached. The reinforcing member 15 is a tapered metal wire that has a circular cross section tapered toward the front end. The material of the reinforcing member 15 is not particularly limited and may be a super-elastic alloy such as stainless steel (SUS304) or Ni-Ti alloy.

The reinforcing member 15 extends from the front end of the outer-tube proximal end 17 through the outer-tube middle portion 13 and then through the proximal-end-side guidewire port 25 to reach near the front end of the outer-tube front end 19. In this embodiment, the front end of the reinforcing member 15 is not fixed to either the outer tube 7 or the inner tube 11. Alternatively, the front end of the reinforcing member 15 may be fixed and interposed between the outer tube 7 and the inner tube 11.

Next, the balloon 3 is described in detail.

The balloon 3 in FIG. 2 is slightly expanded from a state in which it is folded at six locations on the circumference of a cross section vertical to the long-axis direction of the catheter 1.

As shown in FIG. 2, the balloon 3 comprises a base portion 3x and a coating agent 3d such as polyvinylpyrrolidone (PVP) and hyaluronic acid coating the surface of the base portion 3x. The base portion has a three-layer structure consisting of a first base layer 3a made of a thermoplastic polyamide elastomer or the like, a second base layer 3b overlaid on the surface of the first base layer and made of another thermoplastic polyamide elastomer or the like, and a third base layer 3c overlaid on the surface of the second base layer 3b and made of a polyamide resin or the like.

As shown in FIG. 2, the entire surface of the base portion 3x of the balloon 3 is irregular. Namely, the entire circumference of a cross section vertical to the long-axis direction of the balloon catheter 1 is irregular. Before the balloon 3 is expanded, however, the surface of the balloon 3 with the coating agent 3d coating the base portion 3x is smooth and flat.

In the base portion 3x according to this embodiment, the surfaces of the first base layer 3a and the second base layer 3b are smooth and flat and the surface of the third base layer 3c is irregular. However, this configuration is not limitative. As long as the surface of the base portion 3x is irregular, the second base layer 3b and the third base layer 3c may be smooth and flat with the first base layer 3a being irregular, or all of the first base layer 3a, the second base layer 3b, and the third base layer 3c may be irregular.

In order to make the surface of the balloon 3 markedly irregular while the balloon 3 is in an expanded state (as described below), however, it is preferable that at least the third base layer 3c is irregular because the third base layer is adjacent to the bottom side of the coating agent 3d.

The irregular profile of the base layers can be formed by various methods that are typically employed for resin film production, such as surface coating (e.g. coating), physical roughening treatment (e.g. sand-matting), and electrical discharge treatment.

In this embodiment, the base portion 3x has a three-layer structure constituted of the first base layer 3a, the second base layer 3b, and the third base layer 3c. Alternatively, the base portion may have a two-layer structure or a one-layer structure.

In consideration of the pressure resistance of the balloon 3 as well as the inflated (expanded) state and the deflated (contracted) state of the balloon 3, however, a three-layer structure as in this embodiment is preferable as of now.

Although the entire circumference of the balloon 3 according to this embodiment of a cross section vertical to the long-axis direction of the catheter 1 is irregular, the surface of the balloon 3 with the coating agent 3d coating the base portion 3x is smooth and flat. Therefore, the balloon 3 can be folded without restraint.

For example, the balloon 3 in FIG. 3 (different from the one in FIG. 2) is folded at four locations on the circumference of a cross section vertical to the long-axis direction of the catheter, in a slightly expanded state.

In this embodiment, the balloon 3 is folded at four or six locations on the circumference of a cross section vertical to the long-axis direction of the catheter 1 as shown in FIG. 2 and FIG. 3. However, this configuration is not limitative. The balloon 3 may be folded in any other configuration without restraint.

Next, the balloon 3 in an expanded state is described below.

When the balloon 3 according to this embodiment is expanded, an irregular region P appears on the surface of the balloon 3 as shown in FIG. 5. In this embodiment, the irregular region P stretches only where the balloon 3 has expanded to its maximum diameter. Alternatively, when it is preferable for production of the balloon 3 to have the irregular region stretch over the entire balloon surface, the irregular region P may stretch over the front-end-fitting member 16 of the balloon 3 and/or the proximal-end-fitting member 12 of the balloon 3 as well.

The balloon 3 according to this embodiment comprises the base portion 3x constituted of the irregular third base layer 3c overlaid on the smooth and flat surfaces of the first base layer 3a and the second base layer 3b and the coating agent 3d coating the surface of the base portion 3x, as described above. When the balloon 3 is expanded, the surface of the balloon 3 becomes irregular together with the coating agent 3d due to the presence of the irregular region of the base portion 3x, as shown in FIG. 6.

In this embodiment, the irregular region P of the balloon catheter 1 stretches where the balloon 3 has expanded to its maximum diameter. Alternatively, the irregular region P may stretch beyond where the balloon 3 has expanded to its maximum diameter to stretch over the front-end-fitting member 16 of the balloon 3 and/or the proximal-end-fitting member 12 of the balloon 3 as described above, or may stretch over any area, such as an area in a balloon catheter according to the second or third embodiment described below.

The irregular region P may have any surface profile without restraint.

Thus, the balloon catheter 1 according to this embodiment comprises the balloon 3, and the balloon comprises the base portion 3x having the irregular region P on the surface and the coating agent 3d coating the surface of the base portion 3x, with the following configuration: before the balloon 3 is expanded, the surface of the balloon 3 is smooth and flat due to the presence of the coating agent 3d; and while the balloon 3 is in an expanded state, the surface of the balloon 3 is irregular due to the presence of the irregular region P of the base portion 3x. This configuration allows restraint-free arrangement of the irregular region P on the balloon 3 for quickly inserting the balloon 3 into a stenosis or stricture or an obstructed part while the balloon 3 is in a contracted state and for securely fixing the balloon 3 within the stenosis or stricture or the obstructed part while the balloon 3 is in an expanded state.

In the case that the irregular region P stretches over the entire circumference of a cross section vertical to the long-axis direction of the balloon catheter 1, a characteristic effect is exhibited that allows easy fixation of the balloon 3 to an affected area regardless of where the affected area is located.

In addition, the balloon 3 of the balloon catheter 1 according to this embodiment, which allows quick insertion of the balloon 3 into a stenosis or stricture or an obstructed part while the balloon 3 is in a contracted state as well as secure fixation of the balloon 3 within the stenosis or stricture or the obstructed part while the balloon 3 is in an expanded state, can be easily produced.

In addition, the balloon 3 of the balloon catheter 1 according to this embodiment has a smooth and flat surface and therefore the balloon 3 can be folded without restraint, namely without requiring differentiation between the irregular region P and the rest.

Next, the second embodiment of the present invention is described with reference to drawings.

### (Second embodiment)

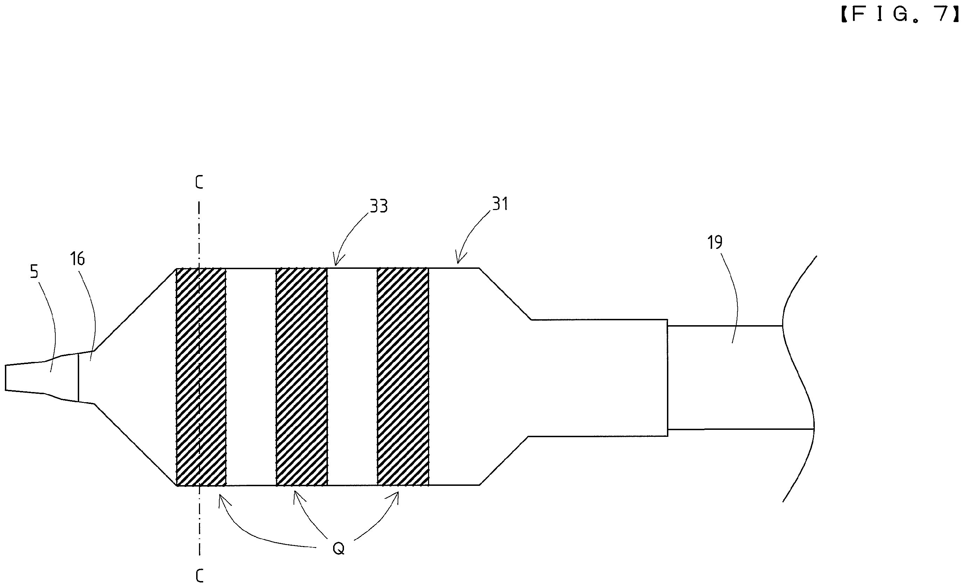

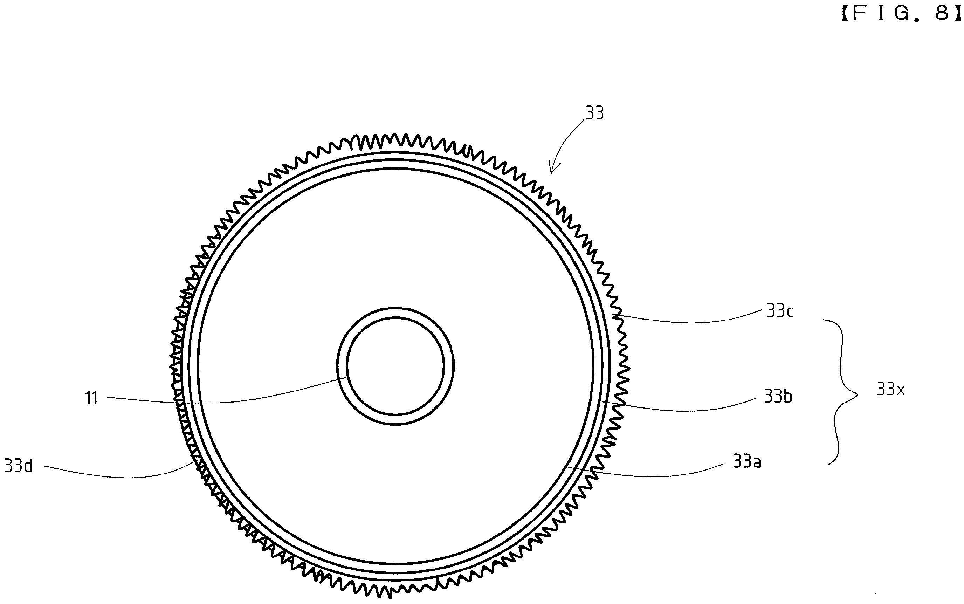

FIG. 7 illustrates an irregular region of the balloon catheter according to the second embodiment (the balloon in the figure is in an expanded state), and FIG. 8 is a sectional view taken from line C-C of FIG. 7.

The following description of this embodiment only includes the differences from the first embodiment, and does not include information that is common to both this embodiment and the first embodiment.

Referring to FIG. 7, a balloon catheter 31 is substantially the same as the balloon catheter 1 except for the balloon.

An irregular region Q of a balloon 33 is different from the irregular region P of the balloon 3. More specifically, the irregular region P of the balloon 3 stretches over the entire circumference of a cross section vertical to the long-axis direction of the balloon catheter 1 and stretches in the long-axis direction of the balloon catheter 1 over the entire area where the balloon has expanded to its maximum diameter, while the irregular region Q of the balloon 33 stretches over the entire circumference of a cross section vertical to the long-axis direction of the balloon catheter 31 as shown in FIG. 8 and is formed at multiple regions (three regions in this embodiment) regularly spaced in the long-axis direction of the balloon catheter 31 as shown in FIG. 7.

The balloon 3 according to the first embodiment comprises the base portion 3x constituted of the irregular third base layer 3c overlaid on the smooth and flat surfaces of the first base layer 3a and the second base layer 3b and the coating agent 3d coating the surface of the base portion 3x, with the following configuration: before the balloon 3 is expanded, the surface of the balloon 3 is smooth and flat due to the presence of the coating agent 3d; and when the balloon 3 is expanded, the irregular region P of the base portion 3x raises the coating agent 3d and thereby the surface of the balloon becomes irregular.

On the other hand, the balloon 33 according to the second embodiment comprises a base portion 33x constituted of an irregular third base layer 33c overlaid on the smooth and flat surfaces of a first base layer 33a and a second base layer 33b and a coating agent 33d coating the surface of the base portion 33x, with the following configuration: before the balloon 33 is expanded, the surface of the balloon 33 is smooth and flat due to the presence of the coating agent 33d (as in FIG. 2 and FIG. 3); and when the balloon 33 is expanded, the irregular region Q of the base portion 33x breaks and protrudes from the surface of the coating agent 33d and thereby the surface of the balloon becomes irregular.

The balloon 33 according to this embodiment in a contracted state is not directly described but is the same as the corresponding state in the first embodiment. The balloon 33 can be folded at four or six locations on the circumference of a cross section vertical to the long-axis direction of the catheter 31 as shown in FIG. 2 and FIG. 3, and can also be folded in any other configuration without restraint.

While the balloon 33 is in a contracted state, the surface of the balloon 33 is smooth and flat due to the presence of the coating agent 33d.

In the balloon catheter 31 according to the second embodiment, when the balloon 33 is expanded, the irregular region Q of the base portion 33x breaks the surface of the coating agent 33d and thereby the surface of the balloon 33 becomes irregular. Therefore, fixation of the balloon 33 to an affected area can become even easier.

Next, the third embodiment of the present invention is described with reference to drawings.

### (Third embodiment)

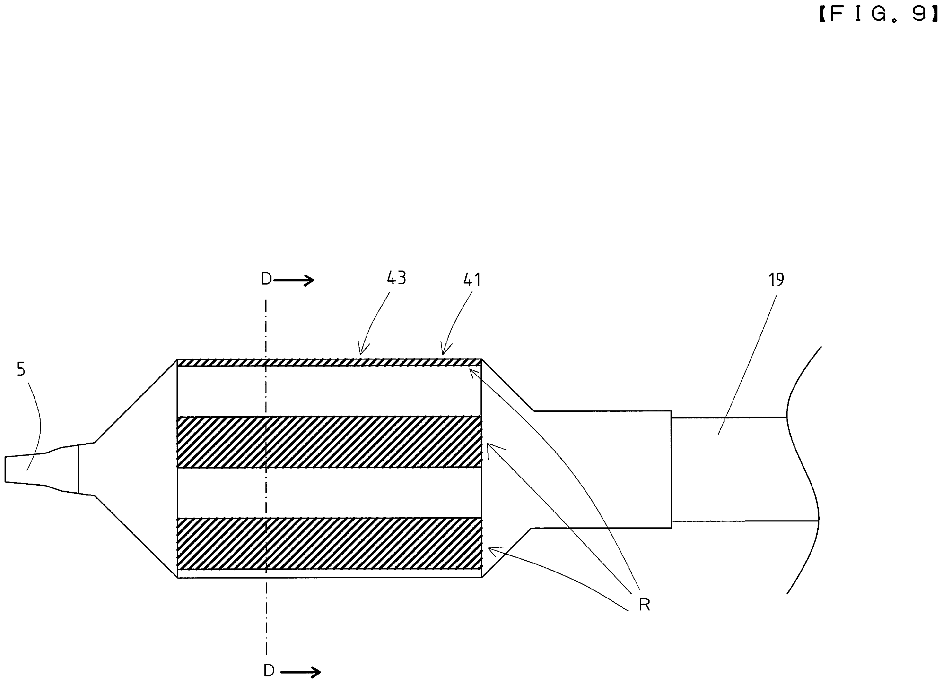

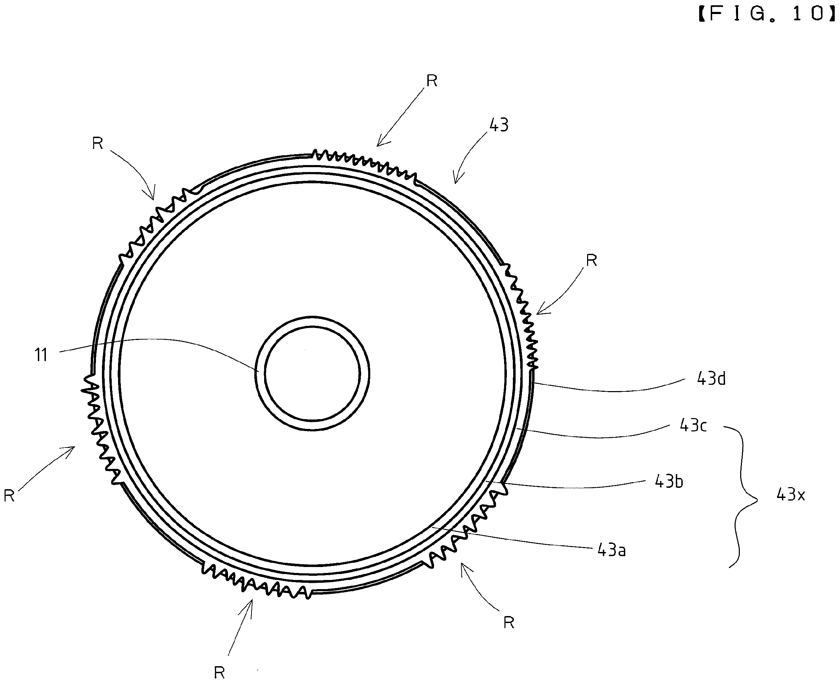

FIG. 9 illustrates an irregular region of the balloon catheter according to the third embodiment (the balloon in the figure is in an expanded state), and FIG. 10 is a sectional view taken from line D-D of FIG. 9.

The following description of this embodiment only includes the differences from the first embodiment, and does not include information that is common to both this embodiment and the first embodiment.

Referring to FIG. 9, a balloon catheter 41 is also substantially the same as the balloon catheter 1 except for the balloon.

An irregular region R of a balloon 43 is different from the irregular region P of the balloon 3. More specifically, the irregular region P of the balloon 3 stretches over the entire circumference of a cross section vertical to the long-axis direction of the balloon catheter 1 and stretches in the long-axis direction of the balloon catheter 1 over the entire area where the balloon has expanded to its maximum diameter, while the irregular region R of the balloon 43 stretches over parts of the circumference of a cross section vertical to the long-axis direction of the balloon catheter 41 as shown in FIG. 10 (six regions in this embodiment) and stretches over the entire area where the balloon has expanded to its maximum diameter in the long-axis direction of the balloon catheter 41 as shown in FIG. 9.

The balloon 3 according to the first embodiment comprises the base portion 3x constituted of the irregular third base layer 3c overlaid on the smooth and flat surfaces of the first base layer 3a and the second base layer 3b and the coating agent 3d coating the surface of the base portion 3x, with the following configuration: before the balloon 3 is expanded, the surface of the balloon 3 is smooth and flat due to the presence of the coating agent 3d; and when the balloon 3 is expanded, the irregular region P of the base portion 3x raises the coating agent 3d and thereby the surface of the balloon becomes irregular.

On the other hand, the balloon 41 according to the third embodiment comprises a base portion 43x constituted of an irregular third base layer 43c overlaid on the smooth and flat surfaces of a first base layer 43a and a second base layer 43b and a coating agent 43d coating the surface of the base portion 43x, with the following configuration: before the balloon 43 is expanded, the surface of the balloon 43 is smooth and flat due to the presence of the coating agent 43d; and when the balloon 43 is expanded, the irregular region R of the base portion 43x breaks and protrudes from the surface of the coating agent 43d and thereby the surface of the balloon becomes irregular.

The balloon 43 according to this embodiment in a contracted state is not directly described but is the same as the corresponding state in the first embodiment. The balloon 43 can be folded at four or six locations on the circumference of a cross section vertical to the long-axis direction of the catheter 41 as shown in FIG. 2 and FIG. 3, and can also be folded in any other configuration without restraint.

While the balloon 43 is in a contracted state, the surface of the balloon 43 is smooth and flat due to the presence of the coating agent 43d.

In the balloon catheter 41 according to the third embodiment, when the balloon 43 is expanded, the irregular region R of the base portion 43x breaks the surface of the coating agent 43d and thereby the surface of the balloon becomes irregular. Therefore, fixation of the balloon 43 to an affected area can become even easier.

### Reference Signs List

- 1, 31, 41:: Balloon catheter
- 3, 33, 43:: Balloon
- 5:: Front-end tip
- 7:: Outer tube
- 9:: Connector
- 11:: Inner tube
- 12:: Proximal-end-fitting member
- 13:: Outer-tube middle portion
- 14:: Guidewire port
- 15:: Reinforcing member
- 16:: Front-end-fitting member
- 17:: Outer-tube proximal end
- 18:: Marker
- 19:: Outer-tube front end
- 21:: Guidewire lumen
- 23:: Expansion lumen
- 25:: Proximal-end-side guidewire port
- 27:: Front-end-side guidewire port

## Claims

1. A balloon catheter comprising a balloon, the balloon comprising:
a base layer having an irregular region on a surface thereof; and
a coating layer coating the surface of the base layer, wherein
before the balloon is expanded, a surface of the balloon is smooth and flat due to the presence of the coating layer, and
while the balloon is in an expanded state, the surface of the balloon is irregular due to the presence of the irregular region of the base layer.

2. The balloon catheter according to claim 1, wherein the irregular region of the base layer stretches over the entire circumference of a cross section vertical to a long-axis direction.

3. The balloon catheter according to claim 1 or 2, wherein upon expansion of the balloon, the surface of the coating layer breaks and thereby the surface of the balloon becomes irregular.

## Amended claims

### Amended claims under Art. 19.1 PCT

1. (Amended) A balloon catheter comprising a balloon, the balloon comprising:
a base layer having an irregular region on a surface thereof; and
a coating layer coating the surface of the base layer, wherein
before the balloon is expanded, an entire surface of the balloon is smooth and flat due to the presence of the coating layer, and
while the balloon is in an expanded state, the surface of the balloon is irregular due to the presence of the irregular region of the base layer.

2. The balloon catheter according to claim 1, wherein the irregular region of the base layer stretches over the entire circumference of a cross section vertical to a long-axis direction.

3. The balloon catheter according to claim 1 or 2, wherein upon expansion of the balloon, the surface of the coating layer breaks and thereby the surface of the balloon becomes irregular.

Statement under Art. 19.1 PCT
Claim 1 has been amended to distinctively state that the entire surface of the balloon is smooth and flat due to the presence of the coating layer before the balloon is expanded.

None of Citation 1 (JP2011-513005A), Citation 2 (JP9-501598A), Citation 3 (JP2015-213623A), and Citation 4 (JP2010-537680A) states a characteristic claimed in Claim 1 of this application that is "the entire surface of the balloon is smooth and flat due to the presence of a coating layer before the balloon is expanded".

The invention claimed in Claim 1 of this application has the following configuration: "before the balloon is expanded, the entire surface of the balloon is smooth and flat due to the presence of the coating agent; and while the balloon is in an expanded state, the surface of the balloon is irregular due to the presence of the irregular region of the base layer". With this configuration, the balloon has the following effects: the balloon allows a restraint-free arrangement of an irregular region on the balloon for quickly inserting the balloon into a stenosis or stricture or an obstructed part while the balloon is in a contracted state and for securely fixing the balloon within the stenosis or stricture or the obstructed part while the balloon is in an expanded state; and the balloon can be folded without restraint, namely without requiring differentiation between the irregular region and the rest.
